(19) Europäisches Patentamt
European Patent Office
Office européen des brevets

(11) Publication number: 0 151 515 B1

(12) # EUROPEAN PATENT SPECIFICATION

(45) Date of publication of patent specification: 03.04.91

(51) Int. Cl.5: C07D 307/20, B01J 31/24, B01J 31/28

(21) Application number: 85300220.2

(22) Date of filing: 11.01.85

(54) **Hydroformylation of allyl alcohol.**

(30) Priority: 02.02.84 US 576513

(43) Date of publication of application:
14.08.85 Bulletin 85/33

(45) Publication of the grant of the patent:
03.04.91 Bulletin 91/14

(84) Designated Contracting States:
BE DE FR GB NL

(56) References cited:
EP-A- 0 038 609
DE-A- 2 649 900
US-A- 4 064 145
US-A- 4 139 542
US-A- 4 400 548

JOURNAL OF ORGANIC CHEMISTRY, vol. 45, no. 11, May 1980, pages 2132-2139, Easton, US; C.U. PITTMAN et al.: "Rhodium-catalyzed hydroformylation of allyl alcohol. A potential route to 1,4-Butanediol"

CHEMIKER-ZEITUNG, vol. 101, nos. 7/8, 1977, pages 343-350; B. FELL et al.: "Isomerisierungsfreie hydroformylierung mit Rhodiumcarbonyl/tert. Phosphin-Komplexkatalysatoren, II. Die hydroformylierung von Allylalkoholen"

(73) Proprietor: TEXACO DEVELOPMENT CORPORATION
2000 Westchester Avenue
White Plains, New York 10650(US)

(72) Inventor: Lin, Jiang-Jen
2617 Oak Meadow Drive
Round Rock Texas 78664(US)
Inventor: Knifton, John Frederick
10900 Castskill Trail
Austin Texas 78750(US)

(74) Representative: Wood, Anthony Charles et al
Urquhart-Dykes & Lord 91 Wimpole Street
London W1M 8AH(GB)

EP 0 151 515 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

This invention is related to the addition of hydrogen and carbon monoxide to olefin compounds to obtain hydroxy-substituted cyclic compounds in the presence of a rhodium-containing catalyst and is more particularly related to such an addition conducted in the presence of a bi-solvent system whereby allyl alcohol is converted into 2-hydroxytetrahydrofuran.

2-hydroxytetrahydrofuran is an important intermediate for producing 1,4-butanediol. A number of methods have been discovered for hydroformylating various unsaturated compounds to useful products.

US-A-4 209 467 teaches a low pressure hydroformylation process in which the catalyst is a reaction product of a cobalt carbonyl and a nitrogen-containing heterocyclic compound having an enolic hydroxy group on the carbon atom adjacent to the ring-forming nitrogen atom, e.g. 2-hydroxypyridine. Ordinarily, the pressures employed therein are in the neighbourhood of 1 to 10 MPa (10 to 100 atmospheres). Unsaturated compounds taught as suitable for this hydroformylation process include ethylenically unsaturated hydrocarbons such as ethlene, propylene, butadiene, other ethylenically-unsaturated compounds such as allyl alcohol, and allyl acetate, etc.

Rhodium catalysts are also known for such hydroformylation processes. DE-A-2649900 discloses the production of 2-hydroxytetrahydrofuran (or the tautomeric 4-hydroxybutanal) in the liquid phase by reacting allyl alcohol under hydroformulation conditions with carbon monoxide and hydrogen in the presence of a rhodium carbonyl catalyst and of an essentially non-polar solvent, such as benzene or toluene. US-A-3 980 670 discloses a process for manufacturing methacrylic acid and butyrolactone by hydroformylation of allyl esters of lower carboxylic acids in the presence of rhodium carbonyl complex catalysts followed by oxidation of the resulting formyl compounds with molecular oxygen to produce 4-acetoxy-n-butyric acid and 3-acetoxy-isobutyric acid as the major products, see also DE-A-2 106 243. Unsaturated compounds such as propylene, may be hydroformylated by means of rhodium/triphenylphosphine/carbonyl complexes formed in situ using a special pre-forming step described in US-A-4 400 549.

US-A-4 064 145 and -4 083 882 describe a method for producing tetrahydrofuran and 1,4-butanediol by reacting synthesis gas with allyl alcohol under hydroformylation conditions in the presence of a rhodium carbonyl-phosphine catalyst complex and various inert solvents such as aromatic and aliphatic hydroxylic solvents. In both patents, the allyl alcohol conversion was reported to be 99% and 4-hydroxybutanal was typically obtained in 87 wt% yield. The major by-product was 2-methyl-3-hydroxypropanal (12 wt%). A rhodium catalyst complexed with special bisphosphine mono-oxide ligands is taught as catalyzing the hydroformylation of olefinic compounds in the presence of dimethylformamide solvent according to US-A-4 400 548. In US-A-4 064 145 the product is extracted with a polar solvent which is immiscible with the non-polar reaction medium and in which the product is soluble. However, the use of two phase solvent systems is not disclosed in either reference.

In J.Org.Chem ., 45 (1980), 2132, C.U. Pittman, Jr. disclosed the hydroformylation of allyl alcohol to 4-hydroxybutanal and 3-hydroxy-2-methylpropanal using $HRh(CO)(PPh_3)_3$ and polymer-bound analogues thereof. The selectivity towards normal/branched products was studied as a function of reaction parameters and ligands employed. The highest normal/branched selectivities (80%) were reported with 1,1'-bis-(diphenylphosphino)ferrocene. Benzene and o-xylene were generally used as solvents.

In J. of Mol. Cat ., 11 (1981), 233-246, N.. deMunck reported a heterogeneous gas phase hydroformylation of allyl alcohol using a supported $HRh(CO)(PPh_3)_3$ catalyst. A very high selectivity to 4-hydroxybutyraldehyde (97%) was achieved, but the conversion of allyl alcohol was only about 20%.

JP-A-29412/1976, and 106407/1979, and Chemical Economy of Engineering Review , Vol. 12, No. 9, (1980) disclose the hydroformylation of allyl alcohol using rhodium catalyst in organic solvents, such as benzene and toluene and a diphosphinoalkane. The overall n-/iso-ratio of the products were 86.6/13.4.

In JP-A-84508/1979 and GB-A-1 493 154, a modified Raney catalyst was employed for the hydrogenation of hydroxybutyraldehydes, forming 1,4-butanediol and 3-methyl-1,3-butanediol.

Many of the systems described above lack good conversions of the unsaturated reactant compound and/or good selectivity to the desired product. Further, recovery of expensive rhodium catalysts is a problem in many of these processes. It would be an advance in the art if a method could be devised for hydroformylating compounds such as allyl alcohol while simultaneously solving the conversion, selectivity and catalyst recovery problems noted above. The present invention provides a process for preparing 2-hydroxytetrahydrofuran which comprises hydroformylating allyl alcohol in the liquid, phase by reaction with carbon monoxide and hydrogen in the presence of a rhodium carbonyl catalyst, which is characterized in that the reaction is performed in a bi-solvent system comprising an aromatic hydrocarbon solvent and an amide solvent having the formula

$$R-\overset{O}{\overset{\|}{C}}-NH_2$$

where R is hydrogen or a lower alkyl group of one to four carbon atoms and where the weight ratio of aromatic hydrocarbon solvent to amide solvent is in the range of 1:5 to 5:1, so as to obtain a reaction mixture where, after the reaction, the mixture separates into two immiscible phases.

In general, the components of the hydroformylation reaction mixture, including the inert solvents, allyl alcohols and rhodium catalyst, may be added in any sequence as long as good agitation is employed to provide a good dispersion or a homogeneous reaction mixture. For example, the following represent some variations in the addition of catalyst components, inert solvents and allyl alcohol addition that can be made without departing from the inventive process. These modifications include

1. The catalyst may be preformed and added to the reaction solvents before addition of the allyl alcohol and other inert solvent components.
2. Alternatively, to minimize catalyst-stability problems, the catalyst can be formed in situ, usually by mixing the inert solvents and allyl alcohol, followed by the addition of the catalyst components to form the reaction mixture.
3. After using either variation 1 or 2, the deoxygenated catalyst-containing reaction mixture is pressurized with CO and hydrogen and heated until the hydroxytetrahydrofuran product is formed.

A rhodium catalyst is used in the present invention. Any rhodium-containing compound capable of forming a carbonyl under the reaction conditions can be used. This rhodium compound may be a carbonyl such as hexarhodium hexadecylcarbonyl. Preferably, the rhodium carbonyl is complexed with a phosphine ligand. Such catalysts are described in US-A- 4 064 145; 4 400 548 and 4 400 549. It is especially preferred that the catalyst be a rhodium carbonyl triphenylphosphine complex catalyst such as hydridocarbonyltris-(triphenylphosphine)rhodium(I), $HRh(CO)(PPh_3)_3$, where Ph represents a phenyl group. Preferably, an excess of the phosphine ligand is added to provide triphenylphosphine.

As noted, the novel feature of the invention is the bi-solvent system. Both components of the bi-solvent must be inert with respect to the carbonylation reaction and both must be immiscible with respect to the other. One solvent is an aromatic hydrocarbon compound. Suitable aromatic compounds include benzene, toluene, ortho-xylene, meta-xylene, para-xylene, ethyl benzene and mixed xylenes, as well as mixtures thereof. Higher molecular weight aromatics with three or more alkyl substituents and more than one aromatic nucleus may also be useful in this application. p-xylene is the preferred non-polar aromatic solvent component in which the rhodium catalyst is soluble.

The other solvent, to be immiscible, is a polar compound into which the desired product, 2-hydroxytetrahydrofuran, is easily soluble. In this way, the purpose of the two-phase solvent system is seen; namely, the in-process separation of the desired product from the catalyst, whereby to provide for easy recovery of the expensive rhodium catalyst. In accordance with this invention it has been found that amides having the structure $R \cdot CO \cdot NH_2$ where R is hydrogen or a $C_1$-$C_4$ alkyl group are suitable for this purpose. Preferably, the amide solvent is formamide or acetamide. Apparently, substituents on the nitrogen in compounds such as in the case of diethylformamide reduce the polar quality of the solvent enough to make it miscible with a non-polar solvent such as p-xylene as the examples will show. Thus, solvents such as diethylformamide would be unsuitable for the process of this invention. If the recommendations herein are followed, the mixture will separate cleanly into two phases after the reaction, which will permit easy recovery of the catalyst and the desired product.

The two solvent components of the bi-solvent system of this invention must be present during allyl alcohol carbonylation in weight ratios from 1:5 to 5:1 in order that the product mix cleanly separates into two phases - a rhodium catalyst-rich phase and a 2-hydroxytetrahydrofuran-rich phase. Preferably, where the two solvent components are p-xylene and acetamide, the two solvents should be present in approximately equal amounts by weight.

The temperature range which can be employed for hydroformylation is a variable which is dependent upon experimental factors, including the total pressure, the mole ratio of hydrogen and carbon monoxide used and the concentrations of reactants and catalyst, among other things. Using rhodium carbonyltriphenylphosphine complex as a representative catalyst, an operable range is from 25 to 125° C, and even higher when superatmospheric pressures above 0.75 MPa are employed. A narrower range of 50 to 120° C represents the preferred temperature range when allyl alcohol is hydroformylated.

The pressure range which can be employed for hydroformylation is a variable which is also dependent on the factors mentioned above. Using rhodium carbonyltriphenylphosphine as a representative catalyst, an

operable pressure range is from 0.75 to 35 MPa, and even higher with a mol ratio $H_2/CO$ of 1:1 when a temperature range of from 25 to 125 ° C is employed. A narrower range of from 3.5 to 10.5 MPa represents the preferred pressure range, when the narrower temperature range of 50 to 125 ° C is employed.

The $H_2/CO$ mol ratio may be varied over a range of from 30:1 to 1:30 when suitable temperatures and pressures are employed. A preferred narrower range of hydrogen/ carbon monoxide is from 2:1 to 1:2.

Experimental variables are important in arriving at reaction times. Generally, substantial conversions (90% or higher) of the allyl alcohol to 2-hydroxytetrahydrofuran can almost always be accomplished within 18 hours, with 2 to 6 hours representing the more usual reaction time interval.

Experimental work indicates that an initial mol ratio of 10 mols to 10,000 mols of allyl alcohol per mol of rhodium-containing catalyst complex can be employed in most instances. The minimum ratio of 0.0001 mol of catalyst per mol of allyl alcohol is herein referred to as a "catalytic ratio" or "catalytic amount". Much higher ratios (e.g. 25 mols of substrate per mol of rhodium catalyst complex) are not harmful but are economically unattractive. For this reason the favoured mol ratio is from 50 to 5,000 mols of allyl alcohol per mol of rhodium catalyst complex.

A suggested weight ratio of p-xylene solvent:amide solvent:allyl alcohol reactant is around 1:1:1. Using the process of this invention, at least 90 wt% of the catalyst will be present in the resulting upper, non-polar p-xylene phase and at least 90 wt% of the 2-hydroxytetrahydrofuran product will be present in the lower, polar amide solvent phase.

The hydroformylation product, 2-hydroxytetrahydrofuran, may be isolated by the usual chemical or physical techniques, such as distillation, solvent extraction, or chromatography. Identification is by nuclear magnetic resonance and/or gas-liquid chromatography.

Conversion as defined herein represent the extent to which allyl alcohol is converted into other products. Conversion is expressed as a percentage and is calculated by dividing the amount of allyl alcohol consumed during hydroformylation by the amount of alcohol originally charged and multiplying the quotient by 100. The allyl alcohol conversion in the process of this invention can be at least 90%.

Yield, as defined herein, represents the efficiency in catalyzing the desired hydroformylation reaction relative to other undesired reactions. In this instance hydroformylation to 2-hydroxytetrahydrofuran is the desired conversion. Yield is expressed as a percentage and is the amount of 2-hydroxytetrahydrofuran product formed, divided by the amount of allyl alcohol charged the quotient being multiplied by 100.

Selectivity, as defined herein, is the efficiency in catalyzing a desired hydroformylation reaction relative to all other (undesired) conversion. Selectivity is expressed as a percentage and is the amount of 2-hydroxytetrahydrofuran product formed, divided by the total amount of $C_3$ plus $C_4$ products formed, the quotient being multiplied by 100. Selectivity can be at least 90% for the inventive process.

EXAMPLE 1

A 300 ml stainless steel stirred autoclave was charged with hydridocarbonyltris(triphenylphosphine)-rhodium(I) $HRh(CO)(PPh_3)_3$ (0.046g), triphenylphosphine (1.3g), allyl alcohol (7.0g), p-xylene (7.0g) and acetamide (7.0g). The reactor was purged of air and pressurized to 0.7 MPa with a mixture of carbon monoxide and hydrogen ($CO/H_2$ = 1:1 mol ratio), then was heated to 60 ° C. The pressure was brought up to 5.61 MPa and maintained during the reaction by the addition of $CO/H_2$ mixture (1:1 mol ratio) through a gas cylinder. After 4 hours, the reaction was stopped and the reactor was cooled to room temperature. The excess gas was vented from the reactor, following which 23.0g of a two-layer product solution was recovered.

The top layer (p-xylene rich), (5.0g) contained 1050 ppm of rhodium (ca. 95% of the Rh charged), but only about 4 wt% concentration of 2-hydroxytetrahydrofuran. The bottom layer (product plus acetamide) (18.0g) contained 14.5 ppm Rh (ca. 5% of Rh charged) and 7.7g of 2-hydroxytetrahydrofuran.

Thus the Rh catalyst and 2-hydroxytetrahydrofuran were separated into two different liquid layers. Gas liquid chromatographic analysis of this product mix further showed:

| | |
|---|---|
| Allyl Alcohol Conversion (%) | >90 |
| Selectivity to 2-hydroxytetrahydrofuran (%) | 95 |
| Estimated Yield of 2-hydroxytetrahydrofuran (mol %) | 74 |

Recovery of rhodium in the product solution was essentially quantitative, 95% of the rhodium being in the top (p-xylene-rich) layer.

EXAMPLE 2

Following the procedures of Example 1, the 300 ml autoclave was charged with $HRh(CO)(PPh_3)_3$ - (0.046g), triphenylphosphine (1.3g), allyl alcohol (7.0g), p-xylene (7.0g) and formamide (7.0g). Reaction with carbon monoxide and hydrogen ($CO/H_2$, 1:1) was conducted at 60°C and 5.61 MPa for 4 hours. After cooling the reactor and depressurizing, a total of 22.7g of two-phase liquid product was recovered. Analysis showed the top layer, rich in p-xylene, comprises 6.5g and contained 620 ppm rhodium but only about 2% concentration of 2-hydroxytetrahydrofuran (0.2g). The bottom layer (16.5g) was found to contain 8.8 ppm of rhodium and 7.4g of 2-hydroxytetrahydrofuran. Gas-liquid chromatography analysis of the total product mix showed:

| | |
|---|---|
| Estimated Allyl Alcohol Conversion (%) | 92 |
| Selectivity to 2-hydroxytetrahydrofuran (%) | 95 |
| Estimated Yield of 2-hydroxytetrahydrofuran (mol %) | 71 |

Rhodium recovery in the product solution was essentially quantitative.

EXAMPLE 3

Following the procedures of Example 1, the 300 ml autoclave was charged with $HRh(CO)(PPh_3)_3$ - (0.046g), triphenylphosphine (1.3g), allyl alcohol (7.0g), toluene (7.0g) and acetamide (7.0g). Reaction with carbon monoxide and hydrogen ($CO/H_2$, 1:1) was conducted at 60°C and 5.61 MPa for 4 hours. After cooling the reactor and depressurizing, a total of 22.7g of two-phase liquid product was recovered. Analysis showed the top layer, rich in toluene, comprises 2.7g and contained 1080 ppm of rhodium but only about 7% concentration of 2-hydroxytetrahydrofuran (~0.2g). The bottom layer (20.0g) was found to contain 56.9 ppm of rhodium and 7.4g of 2-hydroxytetrahydrofuran. The proton-NMR analysis of the total product mix showed:

| | |
|---|---|
| Estimated Allyl Alcohol Conversion (%) | >95 |
| Selectivity to 2-hydroxytetrahydrofuran (%) | 84 |
| Estimated Yield of 2-hydroxytetrahydrofuran (mol %) | 71 |

EXAMPLE 4

Following the procedures of Example 1, the 300 ml autoclave was charged with hexarhodium hexadecacarbonyl (0.0089g), triphenylphosphine (1.3g) allyl alcohol (7.0g), p-xylene (7.0g) and acetamide (7.0g). Reaction with carbon monoxide and hydrogen ($CO/H_2$, 1:1) was conducted at 60 °C and 5.61 MPa for 4 hours. After cooling and depressurizing the reactor, a total of 23.5g of two-phase liquid product was recovered. Analysis showed the top layer, rich in p-xylene, comprised 4.5g and contained 301 ppm of rhodium but only a trace amount of unreacted allyl alcohol. The bottom layer (19.0g) was found to contain 46.5 ppm of rhodium, 2.4g of 2-hydroxytetrahydrofuran and 4.6g of unreacted allyl alcohol. The proton-nuclear magnetic resonance analysis of the total product mix showed:

| | |
|---|---|
| Estimated Allyl Alcohol Conversion (%) | 34 |
| Selectivity to 2-hydroxytetrahydrofuran (%) | 73 |
| Estimated Yield of 2-hydroxytetrahydrofuran (mol %) | 23 |

COMPARATIVE EXAMPLE 5

The reaction mixture was $HRh(CO)(PPh_3)_3$ (0.042g), $Ph_3P$ (1.3g), allyl alcohol (7.0g), p-xylene (7.0g) and diethylformamide (7.0g). Similar reaction conditions to those of Example 1 were used. At the end of the reaction, gas-liquid chromatography analysis showed a 93% yield of 2-hydroxytetrahydrofuran was obtained, but only a single-phase homogeneous product solution was observed.

COMPARATIVE EXAMPLE 6

The identical procedures were used as in Example 1 except that the following quantities were used: $HRh(CO)(PPh_3)_3$ (0.046g), $Ph_3P$ (1.3g), allyl alcohol (10g), p-xylene (10g) and acetamide (1.0g). After 4 hour reaction, gas-liquid chromatography analysis showed a 96% yield of 2-hydroxytetrahydrofuran was obtained. The rhodium catalyst and the product were in a single homogeneous solution. This Example demonstrates the need for relatively equal amounts of each of the two polar and non-polar solvents.

## Claims

1. A process for preparing 2-hydroxytetrahydrofuran which comprises hydroformylating allyl alcohol in the liquid phase by reaction with carbon monoxide and hydrogen in the presence of a rhodium carbonyl catalyst, characterized in that the reaction is performed in a bi-solvent system comprising an aromatic hydrocarbon solvent and an amide solvent having the formula

$$R-\overset{\overset{O}{\|}}{C}-NH_2$$

where R is hydrogen or a lower alkyl group of one to four carbon atoms and where the weight ratio of aromatic hydrocarbon solvent to amide solvent is in the range of 1:5 to 5:1, so as to obtain a reaction mixture where, after the reaction, the mixture separates into two immiscible phases.

2. A process according to claim 1 characterized in that the aromatic hydrocarbon solvent is p-xylene.

3. A process according to claim 1 characterized in that the amide solvent is formamide or acetamide.

4. A process according to any of the preceding claims characterized in that the catalyst is a rhodium carbonyltriphenylphosphine complex together with excess triphenylphosphine.

5. A process according to any of claims 1 to 3 characterized in that the catalyst is hydridocarbonyltris-(triphenylphosphine)rhodium(I).

6. A process according to any preceding claims characterized in that the reaction is performed at a temperature from 50° to 120° C and at a pressure in the range of from 3.5 to 10.5 MPa.

## Revendications

1. Procédé de préparation du 2-hydroxytétrahydrofuranne, qui comprend l'hydroformylation de l'alcool allylique en phase liquide, par réaction avec du monoxyde de carbone et de l'hydrogène, en présence d'un catalyseur au rhodium-carbonyle, caractérisé en ce que la réaction est mise en oeuvre dans un système à deux solvants comprenant un solvant de type hydrocarbure aromatique et un solvant de type amide de formule

$$R-\overset{\overset{O}{\|}}{C}-NH_2$$

dans laquelle R est un hydrogène ou un groupe alkyle inférieur d'un à quatre atomes de carbone et où le rapport pondéral du solvant de type hydrocarbure aromatique au solvant de type amide est dans l'intervalle de 1:5 à 5:1, de manière à obtenir un mélange réactionnel dans lequel, après la réaction, le mélange se sépare en deux phases non miscibles.

2. Procédé selon la revendication 1, caractérisé en ce que le solvant de type hydrocarbure aromatique est le p-xylène.

3. Procédé selon la revendication 1, caractérisé en ce que le solvant de type amide est le formamide ou l'acétamide.

4. Procédé selon l'une quelconque des revendications précédentes, caractérisé en ce que le catalyseur est un complexe rhodium-carbonyle-triphénylphosphine avec un excès de triphénylphosphine.

5. Procédé selon l'une quelconque des revendications 1 à 3, caractérisé en ce que le catalyseur est l'hydridocarbonyltris(triphénylphosphine)-rhodium(I).

6. Procédé selon l'une quelconque des revendications précédentes, caractérisé en ce que la réaction est mise en oeuvre à une température dans l'intervalle de 50° à 120° C et sous une pression dans l'intervalle de 3,5 à 10,5 MPa.

## Ansprüche

1. Verfahren zur Herstellung von 2-Hydroxytetrahydrofuran, welches die Hydroformylierung von Allylalkohol in der flüssigen Phase durch Reaktion mit Kohlenmonoxid und Wasserstoff in Gegenwart eines Rhodium-Carbonyl-Katalysators umfaßt, dadurch gekennzeichnet, daß die Reaktion in einem Zwei-Lösungsmittel-System durchgeführt wird, das ein aromatisches Kohlenwasserstoff-Lösungsmittel und ein Amid-Lösungsmittel mit der Formel

$$R - \overset{\overset{O}{\|}}{C} - NH_2,$$

in der R Wasserstoff oder eine Niederalkylgruppe mit einem bis vier Kohlenstoffatomen ist, umfaßt, wobei das Gewichtsverhältnis von aromatischem Kohlenwasserstoff-Lösungsmittel zu Amid-Lösungsmittel im Bereich von 1:5 bis 5:1 liegt, um eine Reaktionsmischung zu erhalten, bei der sich nach der

Reaktion die Mischung in zwei unmischbare Phasen trennt.

2. Verfahren nach Anspruch 1, dadurch gekennzeichnet, daß das aromatische Kohlenwasserstoff-Lösungsmittel p-Xylol ist.

3. Verfahren nach Anspruch 1, dadurch gekennzeichnet, daß das Amid-Lösungsmittel Formamid oder Acetamid ist.

4. Verfahren nach einem der vorangehenden Ansprüche, dadurch gekennzeichnet, daß der Katalysator ein Rhodium-Carbonyl-Triphenylphosphin-Komplex zusammen mit überschüssigem Triphenylphosphin ist.

5. Verfahren nach einem der Ansprüche 1 bis 3, dadurch gekennzeichnet, daß der Katalysator Hydrido-Carbonyltris(triphenylphosphin)rhodium(I) ist.

6. Verfahren nach einem der vorangehenden Ansprüche, dadurch gekennzeichnet, daß die Reaktion bei einer Temperatur im Bereich von 50° bis 120°C und bei einem Druck im Bereich von 3,5 bis 10,5 MPa durchgeführt wird.